# FASCICULE DE BREVET EUROPEEN

(11) **EP 2 727 579 B1**
(45) Date de publication et mention de la délivrance du brevet: **12.12.2018**
(21) Numéro de dépôt: 13305462.7
(22) Date de dépôt: 09.04.2013
(51) Int. Cl.: A61K 8/60, A61K 8/73, A61K 8/99, A61Q 19/08

(54) **Principe actif à application cutanée obtenu à partir de Metschnikowia agaves et utilisations pour améliorer l'état de la peau**
Wirkstoff zur Anwendung auf der Haut, der aus Metschnikowia-Agaven gewonnen wird, und Anwendungen zur Verbesserung des Hautzustands
Active ingredient for dermal application obtained from Metschnikowia agaves and uses for improving skin condition

(30) Priorité: 16.04.2012 FR 1253471
(43) Date de publication de la demande: 07.05.2014
(73) Titulaire: Societe Industrielle Limousine d'Application Biologique, 19130 Objat (FR)
(72) Inventeur: Paufique, Jean, 19130 Objat (FR)
(74) Mandataire: Aquinov

(56) Documents cités:
- WO-A1-03/068243
- FR-A1- 2 797 186
- FR-A1- 2 949 684
- US-B1- 6 875 754
- DATABASE WPI Week 200511 Thomson Scientific, London, GB; AN 2005-095211 XP002689880, -& JP 2005 015348 A (ASAHI DENKA KOGYO KK) 20 janvier 2005 (2005-01-20)
- DATABASE WPI Week 200342 Thomson Scientific, London, GB; AN 2003-443808 XP002689881, -& JP 2002 370960 A (NOEVIR KK) 24 décembre 2002 (2002-12-24)

## Description

La présente invention se rapporte à un principe actif obtenu à partir de *Metschnikowia agaves,* ainsi que son utilisation pour améliorer l'état de la peau en particulier pour lutter contre le vieillissement cutané et améliorer l'âge perçu. L'invention concerne également les compositions cosmétiques incluant ce principe actif, et un procédé de traitement cosmétique destiné à prévenir et/ou lutter contre le vieillissement cutané.

La peau est un organe complexe qui couvre la surface totale du corps et exerce de nombreuses fonctions vitales. Elle est constamment soumise à des agressions, tant externes qu'internes, qui peuvent menacer son équilibre et son aspect.

C'est pourquoi on recherche des principes actifs capables de protéger la peau contre ces agressions susceptibles d'altérer son bon fonctionnement et son aspect, et de lutter contre les manifestations qui en découlent.

En particulier, dans le domaine cosmétique, on recherche des principes actifs pour le traitement de la peau essentiellement afin d'agir sur les propriétés biomécaniques de la peau et de disposer de caractéristiques anti-vieillissement, hydratantes et anti-rides.

Pour répondre à cette problématique, la présente invention vise un principe actif obtenu à partir de *Metschnikowia agaves, à savoir un hydrolysat de Metschnikowia agaves* comprenant des carboydrates, et son utilisation au sein d'une composition à application cutanée destinée au traitement de la peau.

*Metschnikowia agaves Lachance* est une levure appartenant à la classe des Saccharomycetes et à la famille Metschnikowiaceae. De forme longue-ovale, elle apparaît sous la forme de colonies blanches. Son mode de reproduction est végétatif par bourgeonnement.

*Metschnikowia agaves,* ainsi désignée d'après le nom de son hôte, a été isolée à partir de l'agave bleu du Mexique, utilisée pour la production de la téquila. L'utilisation de cette levure dans le domaine cosmétique et/ou dermopharmaceutique n'a jamais été envisagée. D'autres sucres de levures sont déjà utilisés en cosmétiques :
- candida saitoana (FR 2 949 684 SILAB SA),
- saccharomyces cerevisiae (FR 2 797186 SILAB SA ; WO 03/068243 CLEARCOLL PTY LTD & COWDEN WILLIAMS BUTLER ; US 6 875 754 GRIESBACH UTE ET AL et JP 2002 370960 NOEVIR KK),
- une levure du groupe Aureobasidium ( JP 2005 015348 ASAHI DENKA KOGYO KK).

Or, de façon surprenante, *Metschnikowia agaves* présente des propriétés remarquables au niveau de la peau et des phanères.

Avantageusement, l'utilisation d'un hydrolysat de *Metschnikowia agaves* comprenant des carbohydrates permet d'améliorer l'état de la peau, en particulier de prévenir et/ou lutter contre les effets du vieillissement cutané. Selon un avantage particulier, l'utilisation d'un hydrolysat de *Metschnikowia agaves* comprenant des carbohydrates sur la peau, permet notamment d'agir de façon spécifique sur l'acide hyaluronique, l'un des marqueurs incontournable et reconnu pour son importance dans les soins anti-vieillissement existants actuellement. Or, d'autres levures ne permettent pas une telle efficacité.

L'invention vise également les compositions cosmétiques pour application topique comprenant un hydrolysat de *Metschnikowia agaves* comprenant des carbohydrates, ainsi qu'un procédé cosmétique de soin de la peau humaine, destiné à prévenir et/ou lutter contre les effets de âge sur la peau, comprenant l'application topique d'une composition renfermant un principe actif obtenu à partir de *Metschnikowia agaves.*

La présente invention est maintenant décrite en détail.

### UTILISATION

Selon un premier aspect, l'invention vise un principe actif obtenu à partir de *Metschnikowia agaves* pour son utilisation comme principe actif dans une composition à application cutanée, ledit principe actif et/ou ladite composition étant destinés à améliorer l'état de la peau, en particulier à prévenir et/ou lutter contre les effets du vieillissement cutané.

Les principes actifs obtenus à partir de levures peuvent être de deux types. Il s'agit :
- soit de molécules produites à partir de levures ; ce sont seulement les molécules produites par les levures et non les levures en tant que telles qui sont utilisées,
- soit de principes actifs obtenus à partir des levures en tant que telles.

Ces deux types de principes actifs sont différents. Dans le premier cas, les molécules produites par une levure peuvent éventuellement être produites par d'autres types de levures, alors que dans le second cas, le principe actif est totalement dépendant du choix de la levure.

Par « principe actif obtenu à partir de *Metschnikowia agaves*» au sens de la présente invention, on entend un principe actif obtenu à partir de la levure *Metschnikowia agaves* en tant que telle et non une molécule ou un mélange de molécules produites à partir de la levure *Metschnikowia agaves.*

Par « composition à application cutanée » au sens de l'invention on entend toute composition destinée à être appliquée sur la peau, préférentiellement une composition cosmétique.

Avantageusement, un principe actif obtenu à partir de *Metschnikowia agaves* permet en effet d'améliorer l'état de la peau, notamment de prévenir et/ou lutter contre les effets du vieillissement cutané, spécifiquement d'hydrater la peau, de lisser les traits du visage et d'atténuer les rides.

Selon l'invention, un principe actif obtenu à partir de *Metschnikowia agaves* est notamment capable d'assurer une efficacité performante sur un des marqueurs essentiels du vieillissement de la peau, l'acide hyaluronique.

L'acide hyaluronique est le composant le plus abondant de la peau et structure avec le collagène et l'élastine, le tissu de soutien du derme. Grâce à sa forte hydroscopie, il présente une capacité unique à capter et à lier une quantité importante d'eau et à remplir, sous forme d'un gel hydraté, les interstices entre les composants fibrillaires de la matrice extracellulaire. Il garantit ainsi l'hydratation de la peau ainsi que le maintien de sa souplesse et sa plasticité. L'acide hyaluronique est un glycosaminoglycane non sulfaté composé d'unités disaccharides répétées de N-acetylglycosamine et d'acide glucuronique. Sa synthèse est assurée par les hyaluronane synthases (HAS1, HAS2 et HAS3) et sa dégradation par les hyaluronidases.

Au cours du vieillissement cutané, le contenu dermique en acide hyaluronique s'amoindrit, ses enzymes de synthèse diminuent également alors que le taux de ses enzymes de dégradation augmente. Ces changements se traduisent par une diminution du contenu en eau de la peau et la perte de son élasticité et l'apparition de rides.

Selon l'invention, un principe actif obtenu à partir de *Metschnikowia agaves* permet de stimuler la synthèse naturelle :
- de l'enzyme de synthèse HAS2, et
- de l'acide hyaluronique.

Un principe actif obtenu à partir de *Metschnikowia agaves* appliqué sur la peau permet donc de réactiver le mécanisme naturel de synthèse de l'acide hyaluronique pour consolider le matelas dermique, hydrater la peau et défroisser les rides.

Avantageusement, il présente une action ciblée en profondeur qui permet d'améliorer l'état de la peau : la peau est plus hydratée, les traits du visage sont visiblement lissés et les rides atténuées en particulier au niveau de la patte d'oie. Selon un mode de réalisation particulièrement adapté, l'invention vise l'utilisation dans une composition, d'un principe actif obtenu à partir de *Metschnikowia agaves,* tel que décrit en suivant.

### PRINCIPE ACTIF

L'invention concerne un principe actif destiné à une utilisation dans une composition à application cutanée, obtenu à partir de *Metschnikowia agaves.*

Il s'agit d'un hydrolysat de *Metschnikowia agaves.* Par « hydrolysat » on entend tout extrait obtenu à partir de *Metschnikowia agaves,* comprenant au moins une étape d'hydrolyse de *Metschnikowia agaves.*

Préférentiellement, le principe actif selon l'invention comprend des oligosaccharides, encore plus préférentiellement, le principe actif selon l'invention comprend des oligosaccharides de degré de polymérisation compris entre 2 et 17, composés notamment d'oligosaccharides d'a-glucanes et de β-glucanes.

Le principe actif présente préférentiellement une couleur jaune clair.

Il peut se présenter sous forme liquide limpide et peut être défini par au moins une des caractéristiques exposées ci-après, préférentiellement toutes.

### Matières sèches :

Le taux de matières sèches d'un principe actif selon l'invention (mesuré par passage à l'étuve à 105°C en présence de sable d'un échantillon de poids initial donné jusqu'à obtention d'un poids constant) peut être compris entre 20 et 100 g/l préférentiellement entre 35 et 50 g/l.

### Mesure du pH :

Le pH (mesuré par la méthode potentiométrique à température ambiante) peut être compris entre 4 et 5, préférentiellement entre 3,5 et 5,5.

### Carbohydrates :

### Détermination de la teneur en sucres totaux

Le dosage de la teneur en sucres totaux peut être réalisé par la méthode de DUBOIS (DUBOIS M. et al., (1956), Analytical chemistry, 28, n°3 p. 350-356). En présence d'acide sulfurique concentré et de phénol, les sucres réducteurs donnent un composé jaune orangé. A partir d'une gamme étalon, on peut déterminer le taux de sucres totaux d'un échantillon. Préférentiellement la teneur en sucres totaux est comprise entre 9 et 48 g/l, en particulier entre 16 et 24 g/l.

La teneur en sucres totaux est donc supérieure à 9% en poids de matière sèche. Elle peut notamment être comprise entre 32 et 69% en poids de matière sèche.

### Caractérisation de la fraction carbohydrate :

La détermination de la taille des carbohydrates d'un principe actif selon l'invention est réalisée par chromatographie liquide haute performance.

Le chromatogramme obtenu montre la présence de monosaccharides de masse moléculaire inférieure à 180Da et d'oligosaccharides et de polysaccharides de masse molaire comprise entre 180 et 18500Da (degré de polymérisation au maximum 103). Les monosaccharides représentent moins de 37%, préférentiellement entre 25 et 37%, et les oligosaccharides et polysaccharides de masse molaire comprise entre 180 et 18500Da représentent au moins 63%, en particulier entre 63 et 75% d'oligosaccharides de degré de polymérisation compris entre 2 et 42, préférentiellement entre 2 et 17.

Les sucres simples du principe actif selon l'invention sont préférentiellement constitués de glucose et de mannose.

Afin de mieux caractériser les sucres du principe actif selon l'invention, des hydrolyses enzymatiques ont été réalisées avec une α-amylase et une β-glucanase. Ces hydrolyses permettent de mettre en évidence si le glucose est présent sous forme d'α-glucanes et de β-glucanes.

Les analyses montrent que le glucose lié est présent sous forme d'oligosaccharides d'α-glucanes et sous forme d'oligosaccharides de β-glucanes.

La fraction glucidique de la présente invention est constituée de monosaccharides et d'oligosaccharides de degré de polymérisation majoritairement compris entre 2 et 17. L'actif contient du glucose et du mannose. Les oligasaccharides sont composés notamment d'α-glucanes et de β-glucanes.

C'est notamment cette fraction qui confère au principe actif selon l'invention un effet sur l'amélioration de l'état de la peau en particulier pour la lutte contre le vieillissement cutané.

### PROCEDE D'OBTENTION

Le principe actif selon l'invention tel que décrit précédemment est obtenu par un procédé comprenant une hydrolyse.

Un procédé particulièrement adapté comprend au moins la succession des étapes suivantes :
- solubilisation de levures *Metschnikowia agaves* dans une solution aqueuse, et
- hydrolyse.

Préférentiellement, l'hydrolyse est une hydrolyse enzymatique.

Selon un mode de réalisation particulièrement adapté, le procédé comprend au moins la succession des étapes suivantes :
- solubilisation de levures *Metschnikowia agaves* dans une solution aqueuse,
- hydrolyse enzymatique,
- inactivation enzymatique par traitement thermique,
- filtration,
- purification,
- filtration et filtration stérilisante.

Des étapes de désodorisation et de décoloration ou de concentration peuvent être ajoutées.

Les paramètres des différentes étapes doivent être ajustés afin d'obtenir des principes actifs comprenant des oligosaccharides, préférentiellement des oligosaccharides de degré de polymérisation principalement compris entre 2 et 17.

### COMPOSITIONS COSMETIQUES ET PROCEDE COSMETIQUE DE SOIN DE LA PEAU

La présente invention couvre aussi les compositions cosmétiques incluant un principe actif obtenu à partir de *Metschnikowia agaves,* dans différentes formes galéniques, adaptées à l'administration par voie topique cutanée.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux ou poudres. Elles peuvent être plus ou moins fluides et avoir l'aspect d'une crème, d'une lotion, d'un lait, d'un sérum, d'une pommade, d'un gel, d'une pâte ou d'une mousse, ou sous forme solide.

Il peut s'agir de compositions comprenant entre 0,1 et 3% de principe(s) actif(s) obtenu(s) à partir de *Metschnikowia agaves* selon la présente invention.

Ces compositions comprennent, outre l'actif, un milieu physiologiquement acceptable et de préférence cosmétiquement acceptable, c'est-à-dire qui ne provoque pas de sensations d'inconfort inacceptables pour l'utilisateur telles que des rougeurs, tiraillements ou picotements.

Les compositions selon l'invention peuvent contenir comme adjuvant au moins un composé choisi parmi :
- les huiles, qui peuvent être choisies notamment parmi les huiles de silicone, linéaires ou cycliques, volatiles ou non volatiles ;
- les cires, telles que l'ozokérite, la cire de polyéthylène, la cire d'abeille ou la cire de carnauba ;
- les élastomères de silicone,
- les tensioactifs, de préférence émulsionnants, qu'ils soient non ioniques, anioniques, cationiques ou amphotères ;
- les co-tensioactifs, tels que les alcools gras linéaires ;
- les épaississants et/ou gélifiants,
- les humectants, tels que les polyols comme la glycérine ;
- les filtres organiques,
- les filtres inorganiques,
- les colorants, les conservateurs, les charges,
- les tenseurs,
- les séquestrants,
- les parfums,
- et leurs mélanges, sans que cette liste soit limitative.

Des exemples de tels adjuvants sont cités notamment dans le Dictionnaire CTFA (International Cosmetic Ingrédient Dictionary and Handbook publié par le Personal Care Product Council).

Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Ces compositions sont notamment destinées à favoriser à améliorer l'état de la peau, en particulier à prévenir et/ou lutter contre les effets de l'âge sur la peau. L'invention vise à cet effet un procédé cosmétique de soin de la peau humaine, destiné à prévenir et/ou lutter contre les effets de l'âge sur la peau, comprenant l'application topique d'une composition renfermant un principe actif obtenu à partir de *Metschnikowia agaves selon* la présente invention.

### EXEMPLES

Un exemple non limitatif de procédé d'obtention, de principe actif obtenu à partir de *Metschnikowia agaves,* est présenté en suivant, ainsi que des exemples de composition incluant un tel principe actif.

### Exemple 1 : procédé d'obtention du principe actif selon l'invention

Un exemple de procédé d'obtention d'un principe actif selon l'invention, comprend la mise en oeuvre des étapes suivantes :
- solubilisation de levures *Metschnikowia agaves* dans l'eau en milieu basique à raison de 20g/l,
- hydrolyse enzymatique des sucres,
- inactivation enzymatique par traitement thermique,
- filtration et purification par récupération du filtrat,
- concentration,
- filtration et filtration stérilisante.

Le principe actif obtenu présente les caractéristiques suivantes :
- aspect : solution aqueuse limpide
- couleur : jaune clair
- teneur en matières sèches : 40,5g/l
- pH : 4,3
- teneur en sucres totaux : 21,7g/l, soit 53,7% de sucres par rapport à la matière sèche, dont 69% sous forme d'oligosaccharides de degré de polymérisation compris entre 2 et 17 sous forme de glucose et mannose,
- teneur en protéines : 23,5%
- teneur en cendres : 22,8%.

### Exemple 2 : utilisation d'un principe actif selon l'invention dans une crème de jour

| | | |
|---|---|---|
| Phase A. | Eau | QSP 100% |
| | Glycérine | 1% |
| Phase B. | DUB GMS AE (Stéarinerie DUBOIS) | 1,3% |
| | DUB SEG (Stéarinerie DUBOIS) | 1% |
| | Cetearyl alcool | 1,3% |
| | DUB MCT 5545 (Stéarinerie DUBOIS) | 6,2% |
| | Lanol 99 (Seppic) | 4% |
| | Cetyl palmitate | 0,8% |
| | Sophiderm MC30 (Sophim) | 3,4% |
| | Huile de palme (Sictia) | 1% |
| Phase C. | Principe actif selon l'invention (exemple 1) | 3% |
| | Conservateur | 0,7% |

Les quantités indiquées sont données en pourcentage de poids.

Cette émulsion onctueuse, fondante, blanche présente un pH de 5,5. En application topique sur la peau, elle présente une pénétration rapide avec un fini doux.

Elle peut être obtenue par la mise en oeuvre des étapes suivantes :
- mélanger A, chauffer au bain-marie à 80°C sous agitation magnétique,
- mélanger B, chauffer au bain-marie à 80°C sous agitation magnétique,
- émulsionner B dans A sous rotor sator à 1600 tours/min,
- A 30°C, ajouter *C*, dans l'ordre indiqué, sous rotor stator à 1500 tours/minute, et
- laisser refroidir sous rotor sator jusqu'à complète homogénéisation de l'émulsion.

### Exemple 3 : utilisation d'un principe actif selon l'invention dans une crème de nuit

| | | |
|---|---|---|
| Phase A. | Eau | QSP 100 % |
| Phase B. | DUB LAHE (Stéarinerie DUBOIS) | 5% |
| | DUB ZENOATE (Stéarinerie DUBOIS) | 5% |
| | Montanov 202 (Seppic) | 4% |
| | Montanov 14 (Seppic) | 4% |
| | Montanov S (Seppic) | 1% |
| | DUB 340 (Stéarinerie DUBOIS) | 3% |
| | DUB Lilirose (Stéarinerie DUBOIS) | 6% |
| | DUB PIS (Stéarinerie DUBOIS) | 6% |
| | DUB PPH1 (Stéarinerie DUBOIS) | 4% |
| Phase C. | Conservateur | 0,7 % |
| | Principe actif selon l'invention (exemple 1) | 3% |

Les quantités indiquées sont données en pourcentage de poids.

Cette émulsion compacte blanche présente un pH de 5,5. En application topique, elle présente une application douce, avec un fini sec et un effet filmogène.

Elle peut être obtenue par la mise en oeuvre des étapes suivantes :
- chauffer A au bain-marie à 80°C sous agitation magnétique,
- mélanger B, chauffer au bain-marie à 80°C sous agitation magnétique,
- émulsionner B dans A sous rotor sator à 3200 tours/min,
- A 40°C, ajouter *C*, dans l'ordre indiqué, sous rotor stator à 3000 tours/minute,
- laisser refroidir sous agitation.

### Exemple 4 : utilisation d'un principe actif selon l'invention dans un sérum repulpant

| | | |
|---|---|---|
| Phase A. | Eau | QSP 100 % |
| | Viscolam AT64P (Rita) | 3,4% |
| | Glycérine | 6% |
| | Stérol CC5595 (Alpinia) | 3,4% |
| | Simulsol 1292 (Seppic) | 3,4% |
| | Phonoemuls 100 (Phoenix Chemical) | 2,7% |
| | Conservateur | 0,7% |
| | Principe actif selon l'invention (exemple 1) | 3% |

Les quantités indiquées sont données en pourcentage de poids.

Ce gel onctueux, transparent présente un pH de 7. Il s'étale facilement, présente une pénétration rapide avec un fini sec et doux.

Il peut être obtenu par la mise en oeuvre des étapes suivantes :
- mélanger A, dans l'ordre sous agitation mécanique à 2000 tours/minute,
- laisser sous agitation mécanique jusqu'à complet refroidissement.

### Exemple 5 : utilisation d'un principe actif selon l'invention dans une mousse couvrante

| | | |
|---|---|---|
| Phase A. | Eau | QSP 100% |
| | Carbopol ETD2050 (Noveon) | 0,7% |
| Phase B. | AAB2 (Aiglon) | 2% |
| | Stéarine TP micronisée (Stéarinerie Dubois) | 2% |
| | DUB GMS AE (Stéarinerie Dubois) | 2% |
| | DUB RG AE (Stéarinerie Dubois) | 2,7% |
| | DUB IPM (Stéarinerie Dubois) | 3% |
| Phase C. | Conservateur | 0,7% |
| | Principe actif selon l'invention (exemple 1) | 3% |
| Phase D. | Titanium dioxide | 3,4% |
| | Kaolin | 2% |

Les quantités indiquées sont données en pourcentage de poids.

Ce gel émulsionné, mousseux, blanc présente un pH de 5,8. En application topique, il présente un étalement confortable avec un fini cotonneux légèrement filmogène.

Il peut être obtenu par la mise en oeuvre des étapes suivantes :
- mélanger A, chauffer au bain marie à 80°C, sous agitation mécanique en veillant à bien disperser le gel (environ 800 tours/minute),
- mélanger B, chauffer au bain-marie à 80°C sous agitation magnétique,
- émulsionner B dans A sous rotor stator à 2200 tours/min,
- ajouter *C*, aussitôt dans l'ordre indiqué sous rotor stator à 2200 tours/min,
- laisser refroidir sous agitation,
- mélanger la phase pulvérulente D à l'aide d'un mortier,
- à 30°C, ajouter D, petit à petit et sous agitation mécanique à 2000 tours/min, en veillant à sa complète dispersion,
- laisser la mousse obtenue sous agitation mécanique jusqu'à sa complète homogénéisation.

### Exemple 6 : utilisation d'un principe actif selon l'invention dans un fluide régénérant

| | | |
|---|---|---|
| Phase A. | Eau | QSP 100% |
| | Glycérine | 4% |
| | Blanose 7M31CF (Hercules) | 0,8% |
| Phase B. | DUB RG AE (Stéarinerie DUBOIS) | 3% |
| | DUB PPH1 (Stéarinerie DUBOIS) | 6,7% |
| | DUB MDIS (Stéarinerie DUBOIS) | 6,7% |
| | DUB MCT5545 (Stéarinerie DUBOIS) | 5,4% |
| Phase C. | Conservateur | 0,7% |
| | Principe actif selon l'invention (exemple 1) | 3% |

Les quantités indiquées sont données en pourcentage de poids.

Ce gel émulsionné, fluide, blanc présente un pH de 7,5. Il présente un étalement confortable, un fini doux et un effet hydraté.

Il peut être obtenu par la mise en oeuvre des étapes suivantes :
- mélanger A, chauffer au bain marie à 80°C, sous agitation mécanique en veillant à bien disperser le gel (environ 1000 tours/minute),
- mélanger B, chauffer au bain-marie à 80°C sous agitation magnétique,
- émulsionner B dans A sous rotor stator à 2500 tours/min,
- à 30°C, ajouter *C* dans l'ordre toujours sous rotor sator à 2200 tours/min,
- laisser refroidir sous agitation jusqu'à complète homogénéisation.

### Exemple 7 : utilisation d'un principe actif selon l'invention dans une émulsion anti-rides

| | | |
|---|---|---|
| Phase A. | Eau | QSP 100% |
| Phase B. | Montanov 68 (Seppic) | 2% |
| | Montanov 202 (Seppic) | 3% |
| | Lanol 99 (Seppic) | 5% |
| | Conservateur | 0,7% |
| Phase C. | Principe actif selon l'invention (exemple 1) | 3% |
| Phase D. | Sepigel 305 (Seppic) | 0,3% |

Les quantités indiquées sont données en pourcentage de poids.

Cette émulsion présente un pH de 6,8.

Elle peut être obtenue par la mise en oeuvre des étapes suivantes :
- chauffer A au bain marie à 80°C,
- mélanger B, dans une marmite et chauffer au bain-marie à 80°C,
- émulsionner A dans B sous rotor-sator entre 2000 et 5000 tours/minute,
- à 50°C, ajouter *C*, puis D, toujours sous rotor sator
- laisser agiter jusqu'à complet refroidissement.

### EVALUATION DE L'EFFICACITE COSMETIQUE D'UN PRINCIPE ACTIF SELON L'INVENTION

### A. Tests in vitro

### Etude de l'effet sur la synthèse de l'acide hyaluronique

Le but de cette étude est d'évaluer l'effet du principe actif de l'exemple 1 sur la capacité à augmenter la synthèse de l'acide hyaluronique.

L'étude a été réalisée sur fibroblastes humains normaux par dosage ELISA, selon le protocole suivant.

A JO, les fibroblastes humains sont ensemencés dans du milieu complet. Les cellules sont ensuite incubées à 37°C dans une atmosphère contenant 5% de *CO*₂. A J2, le milieu de culture est éliminé et remplacé par du milieu contenant le principe actif de l'exemple 1 à 0,5% , 1% et 2% (V/V). Le TGF-β à 10 ng/ml est utilisé comme témoin positif.

Les cellules sont ensuite incubées à 37°C pendant 48H.

Les surnageants sont ensuite récupérés et stockés à -80°C en attente du dosage ELISA.

Les résultats du dosage sont présentés dans le tableau suivant :

**Tableau 1**

| | Synthèse d'acide hyaluronique (ng/*µ*g protéines) | Taux d'acide hyaluronique / Témoin (%) |
|---|---|---|
| Témoin | 1280 | |
| TGF-β 10ng/ml | 2343 | +83 |
| Principe actif exemple 1 0,5% | 1878 | +47 |
| Principe actif exemple 1 1% | 2523 | +97 |
| Principe actif exemple 1 2% | 4309 | +237 |

Ces résultats montrent bien qu'un principe actif obtenu à partir de *Metschnikowia agaves* permet de stimuler la synthèse d'acide hyaluronique dans les cellules dermiques.

En particulier testé à 2% sur fibroblastes humains, le principe actif de l'exemple 1 permet de stimuler la synthèse de l'acide hyaluronique de 237%.

Afin de démontrer que cette efficacité est originale et spécifique de la levure sélectionnée selon l'objet de l'invention, le même essai a été réalisé sur d'autres levures à 1%.

Les résultats obtenus sont présentés dans le tableau 2 ci-dessous :

| Tableau 2 | Taux d'acide hyaluronique / Témoin (%) |
|---|---|
| Principe actif exemple 1 1% | +97% |
| Principe actif obtenu à partir de Saccharomyces cerevisiae 1% | +24% |
| Principe actif obtenu à partir de Candida saitoana à 1% | +15% |

Ces résultats montrent bien que tous les actifs issus de levures ne présentent pas une efficacité identique, et que la levure objet de la présente invention se différencie fortement de levures utilisées en cosmétique que sont Saccharomyces cereviviae et Candida saitoana.

### Etude de l'effet sur l'expression de la hyaluronane synthose-2

L'objectif de cette étude est d'évaluer l'effet d'un principe sur sa capacité à augmenter l'expression des ARNm codant pour la hyaluronane synthase-2 (HAS2), enzyme de synthèse de l'acide hyaluronique.

L'étude a été réalisée par PCR quantitative sur fibroblastes humains normaux, selon le protocole opératoire décrit en suivant.

A JO, les fibroblastes humains normaux sont ensemencés dans du milieu complet et les cellules sont ensuite incubées à 37°C.

A J1, le milieu de culture est éliminé et remplacé par du milieu SVF contenant le principe actif de l'exemple 1 à 0,5% - 1% et 2% (V/V). Le TGF-β à 10ng/ml est utilisé comme témoin positif.

Les cellules sont ensuite incubées à 37°C pendant 24H, les cellules sont récupérées et les ARN totaux extraits.

Les ARN ont été reverse-transcripts et les ADN complémentaires obtenus ont été analysés par la technique de PCR quantitative. Les ARNm de la protéine ribosomale S27, témoin interne de référence, ont été également analysés en parallèle des ARNm de HAS2.

La quantification de l'incorporation de fluorescence (SYBR Green) a été mesurée en continu à l'aide d'un thermocycleur. L'analyse des Ct (quantification relative) est réalisée à l'aide d'un logiciel adapté.

Les résultats du dosage sont présentés dans le tableau suivant :

**Tableau 3**

| | Taux d'ARNm de HAS2 (%) | Efficacité / Témoin (%) |
|---|---|---|
| Témoin | 100 | |
| TGF-β 10ng/ml | 318 | +218 |
| Principe actif exemple 1 0,5% | 252 | +152 |
| Principe actif exemple 1 1% | 460 | +360 |
| Principe actif exemple 1 2% | 751 | +651 |

Ces résultats montrent bien qu'un principe actif obtenu à partir de *Metschnikowia agaves* permet d'augmenter l'expression des ARNm codant pour la hyaluronane synthase-2.

En particulier testé à 2% sur fibroblastes humains, le principe actif de l'exemple 1 permet d'augmenter l'expression des ARNm codant pour la hyaluronane synthase-2 de 651%.

### B. Tests in vivo

### Etude de l'effet hydratant : comparaison à l'acide hyaluronique

L'objectif de cette étude est d'évaluer *in vivo* l'effet hydratant d'un principe actif selon l'invention formulé à 3% (composition de l'exemple 7, comprenant 0,13% de principe actif en poids de matière sèche) en émulsion contre placebo au niveau du visage. L'effet de l'acide hyaluronique, molécule de référence (formulé dans une composition identique à 0,13% en poids de matière sèche), a également été testé dans les mêmes conditions.

L' étude a été réalisée en hémi-visage. Les produits ont été randomisés sur 30 volontaires répartis de la façon suivante :
- l'étude du placebo, 20 volontaires, d'âge moyen 58 ans
- l'étude du principe actif selon l'invention, 20 volontaires, d'âge moyen 59 ans
- l'étude de l'acide hyaluronique, 20 volontaires, d'âge moyen 59 ans

Les mesures du contenu en eau ont été réalisées au niveau du bas du visage à l'aide d'un MoistureMeter-D® avant et après 28 jours d'applications biquotidiennes.

Le MoistureMeter-D® génère une onde électro-magnétique à haute fréquence qui est envoyée par une sonde sur la peau. L'onde électromagnétique réfléchie est enregistrée, on obtient ainsi une constante diélectrique proportionnelle au contenu en eau du tissu mesuré. Plus la valeur de cette constante est élevée plus le contenu en eau du tissu est important. La sonde utilisée pour la réalisation des mesures est la sonde S15 qui mesure principalement le contenu en eau du derme. Le protocole opératoire de l'étude est décrit en suivant.

Entre J-14 et JO, les volontaires appliquent une crème placebo sur l'ensemble du visage.

A JO, les volontaires viennent au laboratoire sans avoir appliqué de produit sur le visage le matin (ni crème, ni maquillage) et on réalise des mesures au MOISTUREMETER-D® du contenu en eau sur chaque zone et on distribue les produits.

Entre JO et J27, les produits sont appliqués deux fois par jour.

A J28, les volontaires viennent au laboratoire sans avoir appliqué de produit sur le visage le matin (ni crème, ni maquillage) et on réalise des mesures au MOISTUREMETER-D® du contenu en eau sur chaque zone.

La moyenne des résultats obtenus sur le contenu en eau, avec le principe actif ou l'acide hyaluronique en pourcentage de ceux obtenus avec le placebo sont présentés dans le tableau 4 ci-dessous.

**Tableau 4**

| | **Variation / Placebo (%)** |
|---|---|
| Principe actif de l'exemple 1 3% | +6,5% |
| Acide Hyaluronique 0,13% | +5,9% |

Ces résultats montrent que dans les conditions de cette étude, après 28 jours d'applications biquotidiennes et en comparaison au placebo, le principe actif selon l'invention formulé à 3% en émulsion améliore significativement l'hydratation de la peau, en augmentant le contenu en eau. En outre, on constate que le principe actif selon l'invention présente un effet comparable à celui obtenu avec un acide hyaluronique de référence formulé à 0,13% et testé dans les mêmes conditions.

### Etude des propriétés anti-rides : comparaison à l'acide hyaluronique

L'objectif de cette étude est d'évaluer *in vivo* l'effet anti-rides d'un principe actif selon l'invention (exemple 1) formulé à 3% (composition de l'exemple 7, comprenant 0,13% de principe actif en poids de matière sèche) en émulsion contre placebo au niveau de la patte d'oie par projection de franges.

L'effet de l'acide hyaluronique, molécule de référence (formulé dans une composition identique à 0,13% en poids de matière sèche), a également été testé dans les mêmes conditions.

L'étude a été réalisée en hémi-visage. Les produits ont été randomisés sur 30 volontaires répartis de la façon suivante :
- l'étude du placebo, 20 volontaires, d'âge moyen 58 ans
- l'étude du principe actif selon l'invention, 20 volontaires, d'âge moyen 59 ans
- l'étude de l'acide hyaluronique, 20 volontaires, d'âge moyen 59 ans

Des acquisitions en 3D par projection de franges de la patte d'oie ont été réalisées avant et après 28 jours de traitement biquotidien.

Les acquisitions ont été réalisées à l'aide d'un appareil de projection de franges dédié à la mesure 3D du relief cutané. Ce système comprend un capteur de mesure associant un projecteur de franges lumineuses et une caméra CCD haute résolution reliée à un logiciel d'acquisition. Un système de repositionnement de la tête du volontaire selon les 3 axes de déplacement permet de retrouver la même zone de mesure aux différents temps de l'étude. L'effet du produit est mesuré sur une région d'intérêt de 15x15mm découpée automatiquement sur l'acquisition originale.

Les paramètres les plus pertinents retenus pour cette étude sont :
- Des paramètres de rugosité en 3D :
   * Sq : moyenne quadratique de rugosité de surface
   * Sa : moyenne arithmétique de rugosité de surface
- Un paramètre de volume :
   *volume négatif : volume inférieur à la surface de la peau

Une diminution de ces différents paramètres est caractéristique d'une amélioration du relief de la surface étudiée et d'une diminution des rides.

Le protocole opératoire de l'étude est décrit en suivant.

Entre J-14 et JO, les volontaires appliquent une crème placebo sur l'ensemble du visage.

A JO, volontaires viennent au laboratoire sans avoir appliqué de produit sur le visage le matin (ni crème, ni maquillage) et on réalise des acquisitions en 3D de la patte d'oie par projection de franges et on distribue les produits.

Entre JO et J27, les produits sont appliqués deux fois par jour.

A J28, les volontaires viennent au laboratoire sans avoir appliqué de produit sur le visage le matin (ni crème, ni maquillage) et on réalise des acquisitions en 3D de la patte d'oie par projection de franges.

La moyenne des résultats obtenus, avec le principe actif ou l'acide hyaluronique en pourcentage de ceux obtenus avec le placebo sont présentés dans le tableau 5 ci-dessous.

**Tableau 5**

| | **Variation / Placebo (%)** | |
|---|---|---|
| | Principe actif exemple 1 3% | acide hyaluronique 0,13% |
| Paramètre Sq | -6,3 % | -5,9 % |
| Paramètre Sa | -7,1 % | -6,3 % |
| Volume négatif | -14,2 % | -10,6 % |

Ces résultats montrent que dans les conditions de cette étude, après 28 jours d'applications biquotidiennes et en comparaison au placebo, le principe actif selon l'invention formulé à 3% en émulsion :
- lisse le relief cutané au niveau des pattes d'oies, puisqu'il diminue le paramètre Sq de 6,3% et le paramètre Sa de 7,1%,
- réduit les rides en permettant une diminution de leur volume (-14,2%).

En outre, on constate que le principe actif selon l'invention présente un effet comparable à celui obtenu avec l'acide hyaluronique, molécule de référence, formulé à 0,13% et testé dans les mêmes conditions, et pour tous les paramètres étudiés.

## Revendications

1. Principe actif destiné à une utilisation dans une composition à application cutanée, **caractérisé en ce qu'**il s'agit d'un hydrolysat de *Metschnikowia agaves* comprenant des carbohydrates.

2. Principe actif selon la revendication 1, **caractérisé en ce qu'**il comprend des oligosaccharides.

3. Principe actif selon l'une des précédentes revendications, **caractérisé en ce qu'**il comprend des oligosaccharides de degré de polymérisation compris entre 2 et 42.

4. Principe actif selon l'une des précédentes revendications, **caractérisé en ce qu'**il comprend des oligosaccharides de degré de polymérisation compris entre 2 et 17 composés d'oligosaccharides d'α-glucanes et d'oligosaccharides de β-glucanes.

5. Principe actif selon l'une des revendications 1 ou 2, **caractérisé en ce que** les oligosaccharides représentent au moins 63% en poids des sucres totaux présents dans le principe actif.

6. Principe actif selon l'une des précédentes revendications, **caractérisé en ce qu'**il se présente sous forme liquide et qu'il présente au moins une des caractéristiques suivantes :
- un taux de matières sèches compris entre 35 et 50g/l,
- un taux de sucres totaux compris entre 16 et 24g/l.

7. Utilisation d'un principe actif selon l'une des précédentes revendications, comme principe actif dans une composition cosmétique à application cutanée, pour améliorer l'état de la peau.

8. Utilisation selon la revendication 7, pour lutter contre le vieillissement de la peau.

9. Utilisation selon la revendication 7 ou 8 pour atténuer les pattes d'oie et/ou à hydrater la peau.

10. Composition cosmétique pour application topique, **caractérisée en ce qu'**elle comprend un principe actif selon l'une des revendications 1 à 6, présent entre 0,1 et 3% en poids total de la composition.

11. Procédé cosmétique de soin de la peau humaine, destiné à prévenir et/ou lutter contre les effets de l'âge sur la peau, comprenant l'application topique d'une composition renfermant un principe actif qui est un hydrolysat de *Metschnikowia agaves* comprenant des carbohydrates.

12. Procédé cosmétique de soin de la peau humaine, destiné à prévenir et/ou lutter contre les effets de l'âge sur la peau, comprenant l'application topique d'une composition selon la revendication 10.

## Patentansprüche

1. Wirkstoff zur Anwendung in einer Zusammensetzung für eine Anwendung auf der Haut, **dadurch gekennzeichnet, dass** es sich um ein Hydrolysat von *Metschnikowia-Agaven* handelt, das Kohlenhydrate enthält.

2. Wirkstoff nach Anspruch 1, **dadurch gekennzeichnet, dass** er Oligosaccharide enthält.

3. Wirkstoff nach einem der vorausgehenden Ansprüche, **dadurch gekennzeichnet, dass** er Oligosaccharide mit einem Polymerisationsgrad zwischen 2 und 42 enthält.

4. Wirkstoff nach einem der vorausgehenden Ansprüche, **dadurch gekennzeichnet, dass** er Oligosaccharide mit einem Polymerisationsgrad zwischen 2 und 17 Verbindungen von α-Glucan-Oligosacchariden und β-Glucan-Oligosacchariden enthält.

5. Wirkstoff nach einem der Ansprüche 1 oder 2, **dadurch gekennzeichnet, dass** der Massenanteil der Oligosaccharide des im Wirkstoff vorhandenen Gesamtzuckers mindestens 63 % beträgt.

6. Wirkstoff nach einem der vorausgehenden Ansprüche, **dadurch gekennzeichnet, dass** er in flüssiger Form vorliegt und mindestens eine der folgenden Eigenschaften aufweist:
- einen Trockenmassenanteil zwischen 35 und 50 g/l,
- einen Gesamtzuckeranteil zwischen 16 und 24 g/l.

7. Anwendung eines Wirkstoffs nach einem der vorausgehenden Ansprüche als Wirkstoff in einer Hautkosmetikzusammensetzung zur Verbesserung des Hautzustands.

8. Anwendung nach Anspruch 7 zur Bekämpfung der Hautalterung.

9. Anwendung nach Anspruch 7 oder 8 zur Abschwächung von Fältchen im Augenbereich und/oder um die Haut mit Feuchtigkeit zu versorgen.

10. Kosmetische Zusammensetzung zur lokalen Anwendung, **dadurch gekennzeichnet, dass** sie einen Wirkstoff nach einem der Ansprüche 1 bis 6 enthält, dessen Massenanteil 0,1 bis 3 % der Zusammensetzung beträgt.

11. Kosmetisches Verfahren für die menschliche Hautpflege, um den Auswirkungen der Alterung der Haut vorzubeugen und/oder diese zu bekämpfen, die lokale Anwendung einer Zusammensetzung umfassend, welche einen Wirkstoff umfasst, der ein Hydrolysat von *Metschnikowia-Agaven* ist und Kohlenhydrate enthält.

12. Kosmetisches Verfahren für die menschliche Hautpflege, um den Auswirkungen der Alterung der Haut vorzubeugen und/oder diese zu bekämpfen, die lokale Anwendung einer Zusammensetzung nach Anspruch 10 umfassend.

## Claims

1. An active principle intended for use in a composition for dermal application, **characterised in that** it is a *Metschnikowia agaves* hydrolysate comprising carbohydrates.

2. The active principle according to claim 1, **characterised in that** it comprises oligosaccharides.

3. The active principle according to either of the preceding claims, **characterised in that** it comprises oligosaccharides with a degree of polymerisation of between 2 and 42.

4. The active principle according to any of the preceding claims, **characterised in that** it comprises oligosaccharides with a degree of polymerisation of between 2 and 17 composed of α-glucan oligosaccharides and β-glucan oligosaccharides.

5. The active principle according to either claim 1 or 2, **characterised in that** the oligosaccharides represent at least 63% by weight of the total sugars present in the active principle.

6. The active principle according to any of the preceding claims, **characterised in that** it is in liquid form and **in that** it has at least one of the following features:
- a solids content of between 35 and 50 g/l,
- a total sugar content of between 16 and 24 g/l.

7. Use of an active principle according to any of the preceding claims, as an active principle in a cosmetic composition for dermal application, for improving the state of the skin.

8. Use according to claim 7, for combating skin aging.

9. Use according to claim 7 or 8 for attenuating crow's feet and/or hydrating the skin.

10. A cosmetic composition for topical application, **characterised in that** it comprises an active principle according to any of claims 1 to 6, present at between 0.1% and 3% by total weight of the composition.

11. A cosmetic method for care of the human skin, intended to prevent and/or combat the effects of age on the skin, comprising the topical application of a composition containing an active principle that is a *Metschnikowia agaves* hydrolysate comprising carbohydrates.

12. A cosmetic method for care of the human skin, intended to prevent and/or combat the effects of age on the skin, comprising the topical application of a composition according to claim 10.
